# EUROPEAN PATENT APPLICATION

(11) **EP 1 961 759 A1**
(43) Date of publication of application: **27.08.2008**
(21) Application number: 07003540.7
(22) Date of filing: 21.02.2007
(51) Int. Cl.: C07K 5/12, A61K 38/07, A61K 38/12, A61P 7/00, A61P 7/02, A61P 35/04, A61P 19/00, A61P 29/00

(54) **Integrin targeted cyclopeptide ligands, their preparation and use**

(71) Applicant: UNIVERSITA' DEGLI STUDI DI MILANO, 20122 Milano (IT)
(72) Inventor: Casiraghi, Giovanni c/o Dipartimento Farmaceutico,, 43100 Parma (IT); Scolastico, Carlo c/o Dipt.di Chimica Org. e Ind., 20133 Milano (IT); Zanardi, Franca, 43100 Parma (IT); Battistini, Lucia, 43100 Parma (IT); Curti, Claudio, 43100 Parma (IT); Rassu, Gloria c/o Consiglio Naz.d. Ricerche,, 07040 LI Punti, Sassari (IT); Auzzas, Luciana c/o Consiglio Nazion.d. Ricerche,, 07040 LI Punti, Sassari (IT); Burreddu, Paola c/o Consiglio Naz.d. Ricerche,, 07040 LI Punti, Sassari (IT); Manzoni,Leonardo,Pierpaolo c/o CISI Centro Interdisc. Studi, 20138 Milano (IT)
(74) Representative: Gislon, Gabriele

(57) **Abstract**

The present invention relates to hybrid cyclopeptide compounds embodying pyrrolidine- or piperidine-based amino acid substructures grafted onto a RGD (-Arg-Gly-Asp-) tripeptide sequence and acting as targeting ligands towards integrin receptors, intended, for example, for the treatment of altered angiogenic phenomena or for the preparation of therapeutically and/or diagnostically useful compounds; the invention also concerns a process for the synthesis of said cyclopeptides and biologically active derivatives thereof.

## Description

### Summary of the invention

The present invention relates to novel hybrid cyclopeptide compounds embodying pyrrolidine- or piperidine-based amino acid substructures grafted onto a RGD (-Arg-Gly-Asp-) tripeptide sequence and acting as targeting ligands towards integrin receptors, intended, for example, for the treatment of altered angiogenic phenomena or for the preparation of therapeutically and/or diagnostically useful compounds; the invention also concerns a process for the synthesis of said cyclopeptides and biologically active derivatives thereof.

### Background of the Invention

Integrins are a family of heterodimeric cell-surface receptor glycoproteins composed of non-covalently associated α and β subunits. The integrin family has expanded in vertebrates and sequencing of the human genome has identified as many as 18 α and 8 β subunits, from which 24 different functional integrins are formed in humans (van der Flier, A.; Sonnenberg, A. Cell. Tissue Res. 2001, 305, 285). Integrins are often described as the "glue of life". This means that the major function of these receptors is maintaining the proper structure of tissues by organization of cell-cell and cell-extracellular matrix tethering. However, despite these structural functions, integrins have been identified as complex signalling engines: their extracellular domains are involved in recognition and binding of the ECM, while their cytoplasmatic tails interact with the cytoskeleton and other intracellular signalling molecules. Current hypotheses suggest that conformational changes resulting from these interactions enable integrins to transmit signals across the membrane in both directions ("outside to inside" and "inside to outside"), resulting in cytoskeleton reorganization (shape change, adhesion, migration), regulation of cell proliferation, and cell survival and apoptosis.

On the whole, the ability of integrins in mediating so many fundamental processes can explain their requirement for the correct development and function of various tissues. Absence of functional integrins is associated with aberrant development of tissues and their functions, ultimately resulting in the initiation and prolongation of many diseases such as thrombosis and related pathologies, tumour formation and metastasis spread, tumour angiogenesis, vascular damage brought about by arteriosclerosis, osteoporosis, various inflammatory disorders and autoimmune diseases.

Hence, it is not surprising that some integrins have become attractive targets for pharmacological intervention in a number of pathological conditions pertaining to thrombotic, inflammatory, immune, degenerative and neoplastic diseases. Medical applications of integrin inhibitors are actively pursued.

In particular, specifically targeted are the sub-receptors αᵥβ₃ and αᵥβ₅ in the field of cancer angiogenesis.

Moreover, and from another relevant point of view, today is well known that the expression of integrin adhesion molecules αᵥβ₃ and αᵥβ₅ on sprouting capillary cells and their interaction with specific matrix ligands play a key role in angiogenesis, the formation of new blood vessels from pre-existing vasculature, that is essential for tumour growth and progression, and formation of metastasis.

The αᵥ integrin receptors are highly expressed on activated endothelial cells and tumour blood vessels, but not expressed in resting endothelial cells and most healthy tissues, making it a potential target for antiangiogenic strategy in cancer treatment.

Inhibition of αᵥ integrin activity by monoclonal antibodies, cyclic RGD peptides, and peptidomimetics has been shown to inhibit angiogenesis and to prevent tumour growth in animal models.

According to another relevant aspect, short peptide sequences of the endogenous integrin ligands that are known to be essential regulators of receptor recognition and activation, or inactivation, guide structural and functional investigations of the integrins, as well as the design of peptides and peptidomimetics as competitive antagonists. A great amount of work, and important results, were generated from the RGD (Arg-Gly-Asp) sequence, a common motif of several endogenous ligands binding integrins that lack the I-domain in α-subunit, among them α_{IIb}β₃ and the αᵥ integrins.

Therefore, the first phase in the planning and development of synthetic antagonists of the αᵥ integrin receptors, was focused on creating a series of low molecular weight peptides where the various and differing amino acid residues flanked the RGD consensus sequence.

The following stage was that of planning peptide or semipeptide analogues possessing a reduced conformational flexibility. These efforts climaxed in the identification and synthesis of effective, conformationally constrained cyclic pseudopeptides such as the antagonist c-RGD-(D-Phe)-N-methyl-V (EMD121974, Cilengitide), which proved to be one of the most potent and selective antagonists for the integrin receptors αᵥβ₃ and αᵥβ₅.

A determining step forward in this important area of research arose from the three-dimensional structural crystallographic resolution of the vitronectin receptor αᵥβ₃ as such and, moreover, of this receptor domain as part of a molecular complex with the EMD 121974 ligand already mentioned above.

Taking heed from this fundamental research, various research groups have introduced, over recent years, numerous peptidomimetic analogues containing the RGD sequence encased between pseudopeptide scaffolds of different form and nature and yet, all isosteric mimetics of the dipeptide structure fV of the lead EMD121974.

A common characteristic of these other compounds is the certain presence of a 15 membered macrocycle, identical to that of EMD121974. This series includes a variety of cyclic RGD pentapeptide analogues incorporating unnatural 6,5- and 7,5-fused 1-aza-2-oxobicycloalkane amino acids such as, for example, ST1646, which displayed one-digit nanomolar dual affinity towards the αᵥβ₃ and αᵥβ₅ integrin receptors.

Another recent fruit of research is the new class of dual ligands towards αᵥβ₃ and αᵥβ₅ integrin receptors which possess a one-digit nanomolar activity (e.g. PRGC013, PRGC008) comparable to that exhibited by the EMD121974 and ST1646 leads. These latest ligands present the structural novelty of pseudopeptide 14-atom macrocycles which are effectively more constrained than classical cyclopentapeptides.

The said prior art agents, however, generally suffer drawbacks deriving from chemical and metabolic lability of the true peptide ligands, and/or from heavy and multi-step syntheses of the pseudopeptide scaffolds to be incorporated into the RGD moiety.

Furthermore, most of the previously disclosed integrin ligands lack functionalities to be exploited as proper anchoring points towards preparation of multivalent architectures or functional ligand-active agent conjugates.

Thus still remains the stringent need of ligands easily obtainable by short and high-yielding syntheses, endowed with firm chemical and metabolic stability, and equipped with exposed anchoring points to be exploited in the construction of biologically useful conjugates, bioconjugates, and multimeric compositions thereof.

### Objects of the invention

It is an object of the present invention to provide novel hybrid cyclopeptide compounds able to selectively bind to integrin receptors and which overcome the drawbacks of the integrin ligands of the prior art.

In particular, an object of the present invention is to provide hybrid cyclopeptide derivatives which are able to selectively bind to integrin receptors with extremely high binding affinity and, in particular, as targeting ligands towards the αᵥβ₃ and αᵥβ₅ integrin receptors, said peptide derivatives are intended for the preparation of therapeutically and/or diagnostically useful compositions.

Said covalent incorporation unexpectedly leads to hybrid cyclopeptide ligands exhibiting low-nanomolar antagonist activity towards the αᵥβ₃ and αᵥβ₅ integrin receptors comparable or optionally superior as compared to the known peptide or pseudo-peptide derivatives.

### Description of the Invention

The above mentioned objects are achieved according to the present invention, through incorporation, within the Arg-Gly-Asp tripeptide sequence, of simple pyrrolidine- or piperidine-based γ-amino acid motifs which are either commercially available or very easily obtainable by synthesis and yet endowed of chemical and metabolic robustness, and equipped with a vacant supplementary nitrogen functionality.

According to one of its aspects, the subject-matter of the present invention is a compound of formula (I) 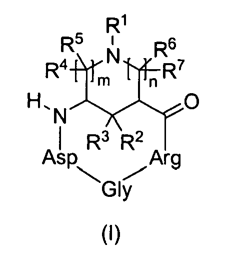 wherein
-Arg-Gly-Asp- corresponds to the sequence of naturally occurring amino acids L-Arginine, Glycine, L-Aspartic Acid, namely the structure of formula (a) 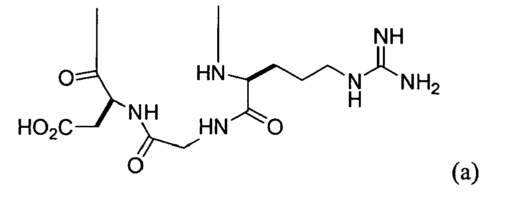
n is 0 or 1;
m is 1 or 2;
provided that when m is 2, n is 0;
R¹ is H, alkyl, aryl, acyl, aroyl, a protective group or a divalent linking moiety;
R², R³, R⁶, R⁷ are independently H, OH, alkoxyl, aryloxyl, alkyl, aryl, acyl, aroyl;
R⁴ and R⁵ are independently H, OH, alkoxyl, aryloxyl, alkyl, aryl, acyl, aroyl, or R⁴ and R⁵ together form an oxo group;

Its salts, racemic mixtures, individual enantiomers, individual diastereoisomers and mixtures thereof in any proportion.

According to another aspect thereof, subject-matter of the invention is a compound of formulae (Ia), (Ib), (Ic), and (Id) 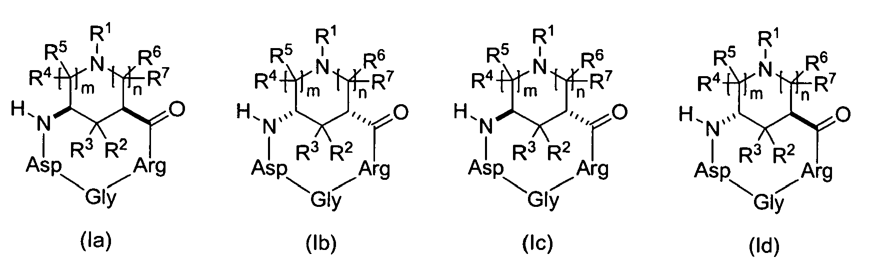 where Arg-Gly-Asp, n, m, R¹, R², R³, R⁴, R⁵, R⁶, and R⁷ are as defined above and the wedge-shaped and dashed bonds indicate that the substituents are positioned above and below the heterocycle plane, respectively.

According to the present invention, the term "alkyl" designates a linear or branched, saturated or unsaturated alkyl group, preferably comprising 1 to 8 carbon atoms. However, it is possible to use alkyl substituents containing a higher number of carbon atoms providing they are compatible with the reaction conditions of the present invention.

Particularly preferred alkyl groups are saturated alkyl chains substituted with NH₂, N₃, OH, or SH functions, optionally substituted with protective groups. Most preferred alkyl substituents are -(CH₂)_{P}CH₂NH₂, -(CH₂)pCH₂N₃, -(CH₂)pCH₂OH, - (CH₂)pCH₂SH, chains wherein p is an integer number between 0 and 10, advantageously between 3 and 5. Also preferred alkyl substituents are polyoxyethylene chains of type -[(CH₂)₂-O]q-CH₂X where q is an integer number between 0 and 10, advantageously between 2 and 3, and X is NH₂, OH, SH or N₃.

According to the present invention, the term "aryl" designates a phenyl group, optionally substituted, or a condensed aromatic group, such as naphthyl or substituted naphthyl.

According to the present invention, the term "acyl" designates a saturated or unsaturated, linear or branched, optionally substituted, alkylcarbonyl group. Preferred acyl groups are those substituted by -C(=O)(CH₂)ₚCH₂NH₂, - C(=O)(CH₂)ₚCH₂N₃, -C(=O)(CH₂)ₚCH₂OH, -C(=O)(CH₂)ₚCH₂SH wherein p is an integer number between 0 and 10, advantageously between 2 and 4.

According to the present invention, the term "aroyl" designates an arylcarbonyl group wherein the aryl component is a phenyl group, optionally substituted, or an heterocyclic aromatic group.

According to the present invention, the term "alkoxyl" designates a saturated or unsaturated, linear or branched, optionally substituted, alkyloxy group.

According to the present invention, the term "aryloxyl" designates an aryloxy group wherein the aryl component is a phenyl group, optionally substituted, or an heterocyclic aromatic group.

According to the present invention, the term "protective group" designates a group suitable to preserve the chemical functionality of the chemical group to which it is bound, specifically the amino or hydroxyl functions. Appropriate protective groups are, for example, benzyl, benzoyl, alkyl, alkanoyl, alkyloxycarbonyl, benzyloxycarbonyl, silyl groups or other substituents routinely used for the protection of such functions, which are well known to those skilled in the art, for example those described in conventional manuals such as Green, T. W.; Wuts, P. G. M. Protective Group in Organic Synthesis (Wiley-Interscience, New York, 1999).

According to the present invention, the term "divalent linking moiety" designates a divalent radical which links the compound of formula (I) to a biologically active moiety. Preferred divalent linking moieties are alkylene or acylene groups, optionally substituted by nitrogen, oxygen, and sulphur functionalities.

In the present description, by "biologically active moiety" is meant any molecule that may be used as a drug, or also as a "targeting" molecule, a diagnostically and/or therapeutically useful compound, such as biologically active moiety, a drug, an imaging detectable moiety, a chelated or polychelated complex of a paramagnetic metal ion, a chelated and polychelated complex of a radionuclide.

Said biologically active molecule may be bound to compound of formula (I), either directly or through an appropriate spacer allowing or promoting binding, and optionally release into the site of action.

The salts of the compounds of formulae (I), (Ia), (Ib), (Ic) and (Id) according to the present invention comprise both those with mineral or organic acids allowing the expedient separation or crystallisation of the compounds of the invention, and those forming physiologically and pharmaceutically acceptable salts, such as for example hydrochloride, hydrobromide, sulphate, hydrogen sulphate, dihydrogen sulphate, maleate, fumarate, 2-naphthalensulphonate, para-toluenesulphonate, oxalate, etc.

Salts of the compounds of formulae (I), (Ia), (Ib), (Ic) and (Id) according to the present invention also further include physiologically and pharmaceutically acceptable quaternary ammonium salts.

Said salts are prepared according to the well known techniques for the person skilled in the art.

The salts of the compounds of the invention also include salts with organic or mineral bases, such as for example alkaline metal or alkaline earth metal salts, such as salts of sodium, potassium or calcium, or amine salts such as trometamol (tromethamine), or salts of arginine, lysine or any other physiologically and pharmaceutically acceptable amine.

According to one preferred embodiment, the subject-matter of the present invention are compounds of formulae (I), (Ia), (Ib), (Ic) and (Id) wherein n is 0.

According to one preferred embodiment, the subject-matter of the present invention are compounds of formulae (I), (Ia), (Ib), (Ic) and (Id) wherein n is 0 and m is 1.

Of course, when n is 0, R⁶ and R⁷ are not present.

According to one preferred embodiment, the subject-matter of the present invention are compounds of formulae (I), (Ia), (Ib), (Ic) and (Id) wherein n is 0, m is 1 and R², R³, R⁴, R⁵, R⁶ and R⁷ are H.

According to one preferred embodiment, the subject-matter of the present invention are compounds of formulae (I), (Ia), (Ib), (Ic) and (Id) wherein n is 0, m is 1, R¹ is H or benzoyl [(C=O)Ph] or propanoyl [(C=O)CH₂CH₃], or (CH₂)₆-NH₂, or (CH₂)₆-N₃, or (C=O)-(CH₂)₅-NH₂, or (C=O)-(CH₂)₅-N₃, or (C=O)-(CH₂OCH₂)p-CH₂NH₂ wherein p is preferably 3 and 4, or (C=O)-(CH₂OCH₂)p-CH₂N₃ wherein p is preferably 3 and 4, R² is H, R³ is H, R⁴ and R⁵ are H, H or together form an oxo group.

According to one preferred embodiment, the subject-matter of the present invention are compounds of formulae (I), (Ia), (Ib), (Ic) and (Id) wherein n is 0, m is 2, R¹ is H or benzoyl [(C=O)Ph] or propanoyl [(C=O)CH₂CH₃], or (CH₂)₆-NH₂, or (CH₂)₆-N₃, or (C=O)-(CH₂)₅-NH₂, or (C=O)-(CH₂)₅-N₃, or (C=O)-(CH₂OCH₂)p-CH₂NH₂ wherein p is preferably 3 and 4, or (C=O)-(CH₂OCH₂)p-CH₂N₃ wherein p is preferably 3 and 4, R² is H, R³ is H, R⁴ and R⁵ are H, H or together form an oxo group.

According to another preferred embodiment, the subject-matter of the present invention are compounds of formulae (I), (Ia), (Ib), (Ic) and (Id) wherein n is 1, m is 1, R¹ is H or benzoyl [(C=O)Ph] or propanoyl [(C=O)CH₂CH₃], or (CH₂)₆-NH₂, or (CH₂)₆-N₃, or (C=O)-(CH₂)₅-NH₂, or (C=O)-(CH₂)₅-N₃, or (C=O)-(CH₂OCH₂)ₚ-CH₂NH₂ wherein p is preferably 3 and 4, or (C=O)-(CH₂OCH₂)p-CH₂N₃ wherein p is preferably 3 and 4, R² is H, R³ is H, R⁴ and R⁵ are H,H or a together form an oxo group, R⁶ and R⁷ are H, H or together form an oxo group.

According to a particular embodiment, another subject-matter of the invention are compounds of formulae (I), (Ia), (Ib), (Ic) and (Id) wherein any spatial arrangement of the R², R³, R⁴, R⁵, R⁶ and R⁷ substituents is possible, that is to say, independently above or under the plane of the nitrogen-containing ring.

A process for the stereoselective synthesis of such compounds is described in detail over the course of the present description, making reference to the synthetic outline reported in the following schemes.

The process for the preparation of the compound of the invention constitutes another subject-matter of the present invention.

We name the nitrogen-containing heterocycle subunits within the general formulae (I), (Ia), (Ib), (Ic) and (Id) with n = 0 and m = 1 the AMPRO (AMinoPROline) subunits, those with n = 0 and m = 2 the AMPIPE (AMinoPIPEcolic) subunits, and those with n = 1 and m = 1 the AMNIPE (AMinoNIPEcotic) subunits.

Scheme 1 outlines the preparation of aminoproline subunits (AMPRO) **1-6, 9,** and **11-14.** As shown in the formulae, compounds **1, 2** and **3** are commercially available as such, while compounds **4, 5, 6, 9, 11, 12, 13** and **14** derive from commercial sources: compounds **4-6** from **3,** compounds **9** and **11** from commercially available cis-4-hydroxy-D-proline **(7),** and compounds **12-14** from **1.**

All the chemistry involved is truly simple and the reactions may be carried out according to techniques known to those skilled in the art. 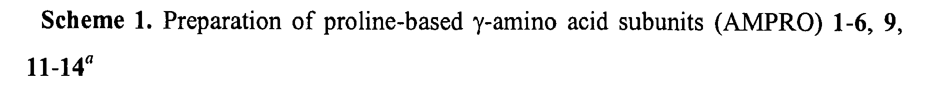 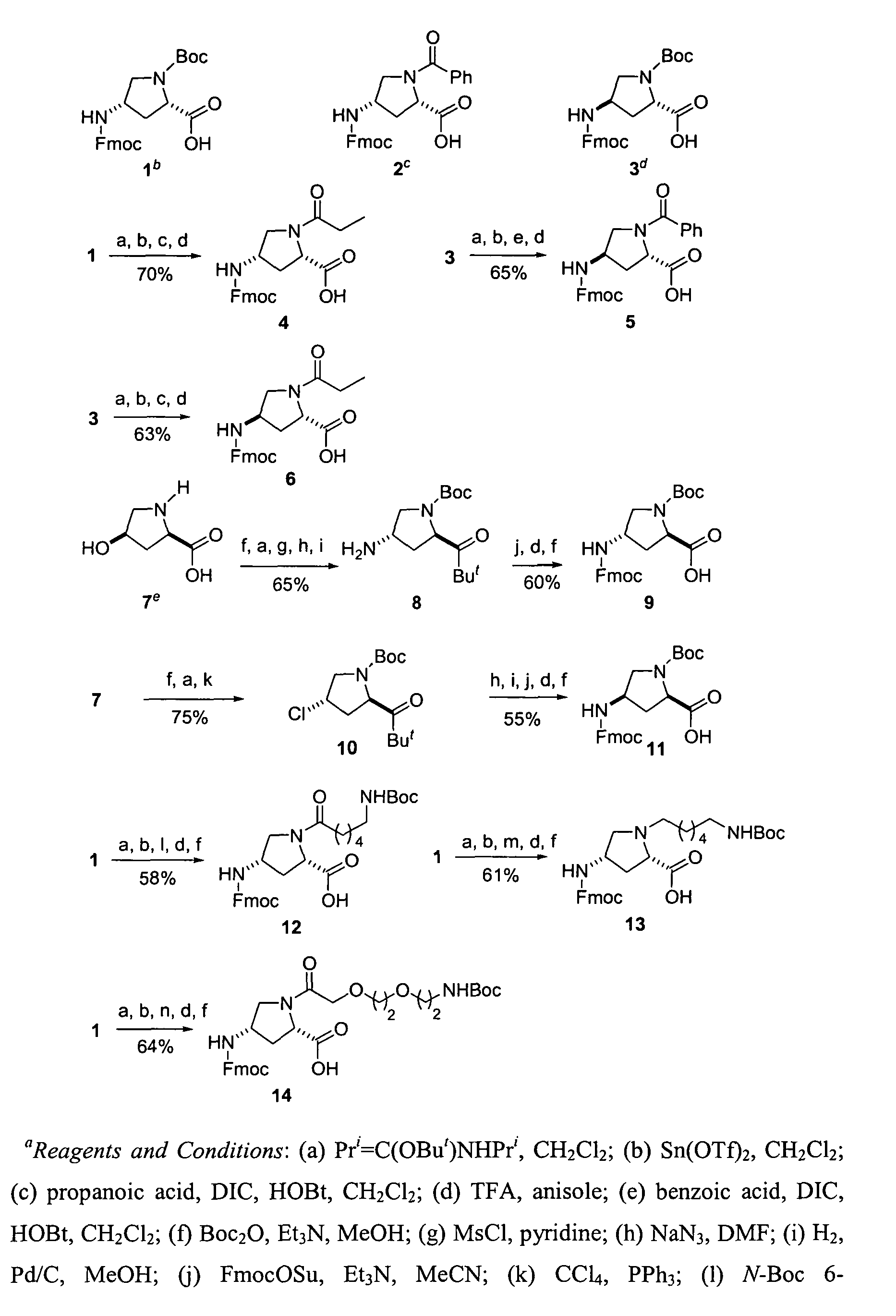 aminohexanoic acid, DIC, HOBt, CH₂Cl₂; (m) CHO(CH₂)₅NHBoc, NaBH(OAc)₃, DCE; (n) NHBoc(CH₂CH₂O)₂CH₂CO₂H, DIC, HOBt, CH₂Cl₂.
*^{b}*Available from NeoMPS, catalogue 2006/2007 (code FA12301); *^{c}*available from NeoMPS, catalogue 2006/2007 (code 12303); *^{d}*available from NeoMPS, catalogue 2006/2007 (code FA12302); *^{e}*available from Aldrich, catalogue 2005/2006 (code H5877).

The synthesis of eleven representative members of the AMPRO-containing RGD-cyclopeptide compound family is illustrated, schematically, in Scheme 2.

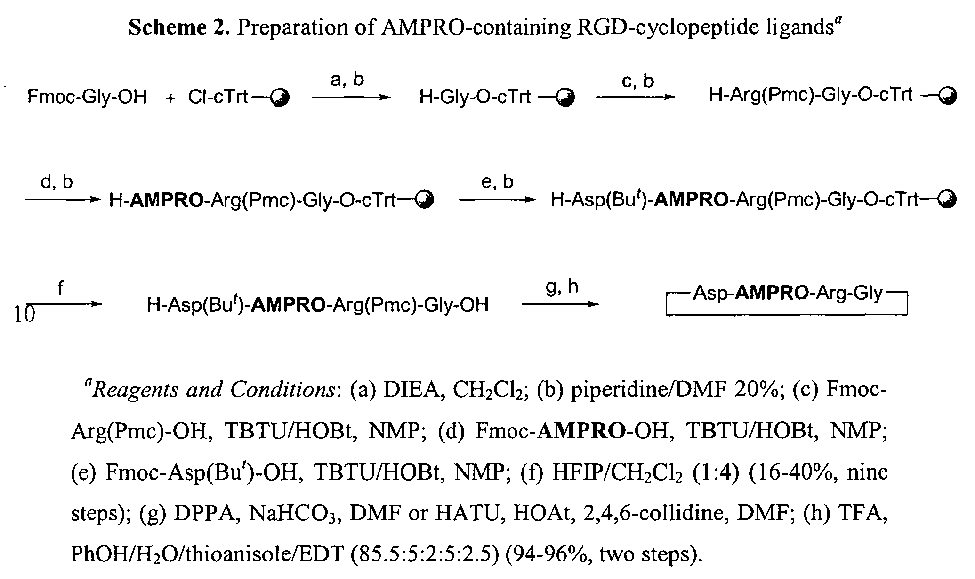

To assemble the collection, we first planned to realize linear tetrapeptide precursors of type H-Asp(Bu*^{t}*)-**AMPRO**-Arg(Pmc)-Gly-OH and then complete the macrocycle via head-to-tail juncture. The AMPRO subunits, 1-6, 9 and 11-14, were critically positioned along the peptide chain, flanked by the aspartate and arginine residues, to create the local constraint that pre-organizes the chain terminals toward macrocyclization. Eleven tetrapeptides of general formula H-Asp(Bu*^{t}*)-**AMPRO-**Arg(Pmc)-Gly-OH were synthesized in parallel on solid phase utilizing a conventional polystyrene-based 2-chlorotrityl chloride resin. Although this type of linker is quite acid-labile, its stability toward bases makes it ideal for the various coupling base promoters as well as for the resident Arg and Asp protecting groups using the Fmoc strategy, the product being readily cleaved from the resin under mild acidic conditions (e.g. hexafluoroisopropanol in dichloromethane).

In the resin loading step, *N*-Fmoc-glycine was attached to the 2-chlorotrityl linker using a 2.5-fold excess amino acid to ensure maximum yield (≥1.55 mmol/g actual loading). After deprotection of the amino group (20% piperidine in DMF), condensation of the Fmoc-Arg(Pmc)-OH residue was attained using the TBTU-HOBt system in the presence of DIEA. Next, the second amino group within the resin-bound dipeptide Fmoc-Arg(Pmc)-Gly-OcTrt was liberated as usual, and an AMPRO platform, chosen among the eleven amino acids of this invention (1-6, 9 and 11-14, Schema 1), was coupled and the resin-bound tripeptide deprotected.

The Fmoc-Asp(Bu*^{t}*)-OH residue was then connected and the amino group liberated to afford a resin-bound tetrapeptide, which was cleaved from the resin with a 1:4 vv mixture of hexafluoroisopropanol in dichloromethane. The crude linear peptides were thus obtained in yields ranging from 16% to 40% for the entire solid phase sequence. The eleven individual linear peptides were cyclized in solution (2.5 mM in DMF) at room temperature using diphenylphosphoryl azidate (DPPA) activation in the presence of solid sodium hydrogen carbonate or the O-(7-azabenzotriazol-1-yl)-*N,N,N',N'-tetramethyluronium* hexafluorophosphate (HATU)/1-hydroxy-7-azabenzotriazole (HOAt) system. After chromatographic purification, the side-chain deprotection was carried out under acidic conditions in the presence of scavengers, with the reagent system TFA, phenol, H₂O, thioanisole, and 1,2-ethanedithiol (EDT). The resulting compounds were first purified by preparative reversed-phase HPLC and finally transformed into stabile hydrochloride salts by exposing the solid materials thus obtained to anhydrous HCl (gas) until constant weight was reached. The global yields of the cyclization/deprotection step ranged from 94 to 96%.

The purity of each macrocycle in the eleven-component collection was checked by two independent RP-HPLC analyses and judged to be ≥98% for all materials. All RGD macrocycles were characterized by high-resolution ESI mass spectrometry as well as various NMR techniques *(see infra).*

Paradigmatic examples of these preparations are detailed in the experimental section.

The structural representation of the eleven AMPRO-containing RGD-structures (Ie)-(Io) is reported in Scheme 3.

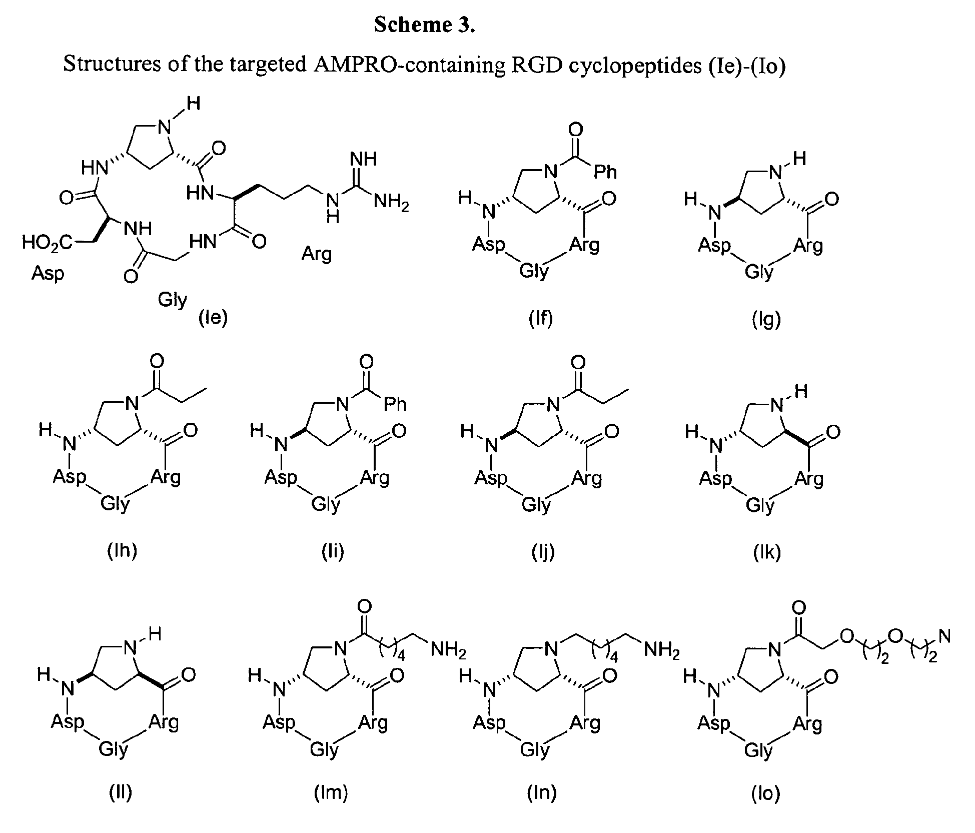

According to the present invention, the 15-membered peptide or pseudo-peptide macrocycle of the known integrin ligands is here replaced by a 14-membered pseudo-peptide, a one-carbon contraction that we found out to be highly beneficial for biological activity. Such a contraction is provided by the conformationally restrained pyrrolidine- or piperidine-based γ-amino acid subunit which bioisosterically replaces the "traditional" dipeptide or dipeptidomimetic moieties.

According to another key aspect of the present invention, the five- or six-membered γ-amino acid moiety within the hybrid cyclopeptides contains a vacant nitrogen heteroatom, prone to be exploited as a conjugation locus for introduction of active moieties and as a useful anchoring point for the introduction of moieties relevant from the pharmacological viewpoint in order to enhance ligand-receptor interactions.

According to another of its aspects, the present invention relates to the use of a compound of formula (I) as a medicament.

Accordingly, a further subject-matter of the present invention is the use of the compounds of general formulae (I), (Ia), (Ib), (Ic) and (Id) for the preparation of drugs, particularly useful for their antagonistic action towards the αᵥβ₃ and αᵥβ₅ integrins.

More precisely, the invention concerns the use of compounds of general formulae (I), (Ia), (Ib), (Ic) and (Id) for the preparation of drugs with antiangiogenic activity, useful for the treatment of altered angiogenic phenomena, such as those encountered in metastasizing tumour processes, retinopathies, acute renal damage and osteoporosis.

According to another of its aspects, the present invention relates to the use of a compound of formula (I) as a diagnostic agent, in particular to be used, but not only, in connection with the diagnosis of tumour-induced angiogenesis and of related disorders.

According to a still different embodiment of the invention, also provided is a method for the treatment of integrin-mediated disorders including, but not limited to, treatment and prevention of tumour-induced angiogenesis and of related disorders, the said method comprising the administration, in a subject in need thereof, of an effective amount of a compound according to the invention.

### Biological Results

The compounds of formulae (I), (Ia), (Ib), (Ic) and (Id) possess extremely interesting biological and pharmacological properties, particularly a marked antagonistic effect towards the αᵥβ₃ and αᵥβ₅ integrin receptors, and display highly appealing antiangiogenic activities.

The ability of the synthesized cyclopeptides to compete with [¹²⁵I]-echistatin for binding to the isolated, purified αᵥβ₃ and αᵥβ₅ receptors was evaluated in a solid-phase receptor assay (Kumar, C. C.; Nie, H.; Rogers, C. P. Malkowski, M.; Maxwell, E.; Catino, J. J.; Armstrong, L. J Pharmacol. Exp. Ther. 1997, 283, 843) and compared with that of known standard binders, EMD121974 (Dechantsreiter, M.A. et al. J. Med. Chem. 1999, 42, 3033; Jonczyk, A. et al. Ger. Offen. DE19534177 A1, 1997), and c(-RGDfV-) (Wermuth, J.; Goodman, S. L.; Jonczyk, A.; Kessler, H. J. Am. Chem. Soc. 1997, 119, 1328).

Integrin-coated 96-well plates were incubated with the ligand in the presence of serially diluted competing compounds. The IC₅₀ ± SD values (nM) were calculated as the concentration of compound required for 50% inhibition of ligand binding as estimated by Allfit program, and are given in Table 1.

These binding assays have been carried out as paradigmatic examples on a selected, yet representative collection of seven novel AMPRO-containing RGD cyclopeptides, namely compounds (Ie), (If), (Ig), (Ih), (Ii), (Ik), (Il) (see Scheme 3).

**Table 1. Binding Affinity of AMPRO-containing ligands (Ie), (If), (Ig), (Ih), (Ii), (Ik), (Il) in radioligand assays at αᵥβ₃ and αᵥβ₅ integrin receptors.^{a}**

| **Cmpd. No.** | **Cmpd. Code** | **Cmpd Label** | **Radioligand Binding Assay** | |
|---|---|---|---|---|
| | | | αᵥβ₃ IC₅₀ (nM) | αᵥβ₅ IC₅₀ (nM) |
| (Ie) | PRGC021 | *c*[-RGD**AMPRO1-**] | 4.03±0.91 | 77±13 |
| (If) | PRGC020 | *c*[-RGD**AMPRO2**-] | 0.34±0.08 | 86±7 |
| (Ig) | PRGC022 | *c*[-RGD**AMPRO3**-] | 7.16±0.96 | 22±3 |
| (Ih) | PRGC023 | *c*[-RGD**AMPRO4**-] | 1.6±0.2 | 11.1±1.2 |
| (Ii) | PRGC025 | *c*[-RGD**AMPRO5**-] | 5.5±0.7 | 14.2±1.0 |
| (Ik) | PRGC026 | *c*[-RGD**AMPR09**-] | 0.9±0.06 | 9.6±0.6 |
| (Il) | PRGC027 | *c*[-RGD**AMPRO11**-] | 7.7±0.04 | 6.56±0.01 |
| | EMD121974 | *c*[-RGDf(NMe)V-] | 18.9±3.1 | 0.13±0.009 |
| | | *c*[-RGDfV-] | 195.9±16.8 | 0.11±0.03 |

| | | | | |
|---|---|---|---|---|
| *^{a}*Average of three or more independent determinations. | | | | |

While the cited specifications illustrate the principles of the present invention, with selected examples reported, it must be intended that the practice of the present invention also includes all usual variations and/or modifications and adaptations as derivable from the claims and intentions of the invention *(vide infra).*

As demonstrated by the results in Table 1, the embodiment of the pyrrolidine scaffold onto the RGD residue resulted in the formation of extremely active ligands reaching one-digit nanomolar binding affinity towards the αᵥβ₃ and αᵥβ₅ integrin receptors. Remarkably, this behaviour favourably compares with the best candidates in this domain as, for example, the previously disclosed hits c[-RGDf(NMe)V-] (EMD121974) and c[-RGDfV-].

According to another favourable aspect, anchoring of differently shaped substituents at the free NH function of the pyrrolidine scaffold was highly tolerated, suggesting that conjugation of active units at this amine point is confidentially viable.

Regarding their use as drugs according to the invention, the compounds of general formulae (I), (Ia), (Ib), (Ic) and (Id), their pharmaceutically acceptable salts, racemic mixtures, individual enantiomers, individual diastereoisomers and mixtures thereof in any proportion, are advantageously formulated into pharmaceutical compositions according to conventional techniques, well known to those skilled in the art.

Thus, according to another aspect thereof, the invention concerns pharmaceutical compositions comprising, as active ingredients, at least one compound of general formulae (I), (Ia), (Ib), (Ic) and (Id), their pharmaceutically acceptable salts, racemic mixtures, individual enantiomers, individual diastereoisomers and mixtures thereof in any proportion, in combination with one or more possible pharmaceutically acceptable carriers or excipients.

The pharmaceutical compositions of the invention are those suitable for oral, rectal, topical, peroral (for example sublingual) and parenteral (for example subcutaneous, intramuscular, intradermal or intravenous) administration. Coated formulations and coated slow-release formulations, as well as transdermal formulation are also encompassed by the present invention.

The compositions of the invention may be prepared according to conventional techniques well known to those skilled in pharmaceutical technique.

In order to obtain a prophylactic or desired therapeutic effect, the dose of active ingredient is advantageously administered in the form of a unit dose, one or more times per day, in an amount which varies depending on the age, the weight and the pathology of the patient. Generally, the compounds of formula (I) may be administered in an amount between 0.1-100 mg per day.

The compounds of formulae (I), (Ia), (Ib), (Ic) and (Id) may be combined with various drugs, for example with a cytotoxic type drug, active towards the tumour pathology.

In fact, the amine functionality of the heterocyclic moiety within the cyclopeptide compounds of the present invention may be exploited as a site for the introduction of groups, relevant from a pharmacological viewpoint, in order to enhance protein-protein or protein-receptor interactions.

Thus, for example, in the compounds of formulae (I), (Ia), (Ib), (Ic) and (Id) wherein R¹ represents a hydrogen atom, the N-H group is available for easy conjugation of a drug. Alternatively, depending on the type of drug to be conjugated, R¹ may be more conveniently a bifunctional group, as defined above.

According to another aspect thereof, the invention concerns the use of compounds of formulae (I), (Ia), (Ib), (Ic) and (Id) as mediators for the transport and release of drugs.

Thus way it is possible to bind a drug, conventionally, through groups available for the formation of a chemical bond as outlined above, to the compounds of formulae (I), (Ia), (Ib), (Ic) and (Id).

In this way, the drugs conjugated to the compounds of formulae (I), (Ia), (Ib), (Ic) and (Id) may then be transported to the desired site of action in order to perform their pharmacological activity.

The conjugates of the compounds of formulae (I), (Ia), (Ib), (Ic) and (Id) as described above, with drugs, advantageously with cytotoxic and antitumour drugs as well as therapeutically and/or diagnostically effective units, constitute another aspect of the present invention.

The skills and relevant features of the present invention will now be described from the experimental viewpoint.

### EXPERIMENTAL SECTION

### GENERAL

All solid-phase synthesis chemicals were dried in dessicator before use; all other chemicals were used as supplied without further purification. Apart from N-methylpyrrolidone (NMP), all organic solvents were dried and freshly distilled before use according to literature procedures. Commercial Fmoc-alfa-amino acids were purchased from Fluka or Bachem; cTrt resin (2-chlorotrityl chloride polymer bound, 100-300 mesh, nominal loading ~1.9 mmol/g, calculated loading 1.55-1.8 mmol/g) was from Fluka, coupling reagents were from either Bachem or Aldrich and all other chemicals and reagents were from either Aldrich or Acros. All moisture sensitive reactions were carried out under a positive pressure of nitrogen or argon. TNBS test was performed according to the following procedure. A few resin beads were sampled and accurately washed with ethanol. The sample was then placed in a vial and 1 drop of a 10% solution of DIEA in DMF and 1 drop of 1% 2,4,6-trinitrobenzenesulfonic acid (TNBS) in DMF were added. The sample was then observed under a microscope and colour changes noted. The TNBS test is considered positive (presence of free amino groups) when the resin beads turn orange or red within 1 min and negative (no free amino groups) when the beads remain colourless.

TLC analysis was performed on silica gel 60 F₂₅₄ plates (Merck) with visualization under short-wavelength UV light or by dipping the plates with molybdate reagent (H₂O/concentrated H₂SO₄/(NH₄)₆Mo₇O₂₄·4H₂O/Ce₂(SO₄)₃ 90/10/25/1 v/v/w/w) followed by heating. Flash chromatography was performed on 40-63 µm silica gel from Merck using the indicated solvent mixtures. Melting points were determined with an optical thermomicroscope Optiphot2-Pol Nikon and are uncorrected. Optical rotations were measured using a Perkin-Elmer model 341 polarimeter at ambient temperature using a 100-mm cell with a 1-mL capacity and are given in units of 10⁻¹ deg cm² g⁻¹.

¹H and ¹³C NMR spectra were recorded on a Bruker Avance 300 operating at 300/75 MHz, or a Varian Mercury Plus MP-400 at 400/100 MHz, or a Varian Inova SB-600 at 600/150 MHz, or a Varian Inova 800 at 800/200 MHz.

Analytical reversed-phase HPLC was performed on a SpectraSystem P2000 apparatus from Thermo Separation Products (UV detection λ = 254 nm) equipped with a Discovery C18-10µm column (250 x 4.6 mm). Semipreparative reversed-phase HPLC was performed on a Prostar 210 apparatus from Varian (UV detection λ = 220 or 254 nm) equipped with a Discovery C18-10µm column (250 x 10 mm). Direct infusion ESI-MS spectra were recorded on Applied Biosystems API 150EX apparatus. High-resolution mass spectrometry (HRMS) measurements were performed on a Bruker APEX IIIQ Fourier transform mass spectrometer equipped with an external electrospray ion source. Elemental analyses were performed by the Microanalytical Laboratory of the University of Parma.

### PREPARATION OF AMPRO-SUBUNITS (SCHEME 1)

**(2*S*,4*S*)-Fmoc-4-Amino-1-Boc-pyrrolidine-2-carboxylic Acid (1)**. The title amino acid was purchased from NeoMPS (catalogue 2006/2007, code: FA12301) and used without further purification.
**(2S,4S)-Fmoc-4-Amino-1-benzoylpyrrolidine-2-carboxylic Acid (2)**. The title amino acid was purchased from NeoMPS (catalogue 2006/2007, code: FA12303) and used without further purification.
**(2*S*,4*R*)-Fmoc-4-Amino-1-Boc-pyrrolidine-2-carboxylic Acid (3)**. The title amino acid was purchased from NeoMPS (catalogue 2006/2007, code: FA12302) and used without further purification.
**cis-4-Hydroxy-D-proline (7).** The title amino acid was purchased from Aldrich (catalogue 2005/2006, code: H5877) and used without further purification. The corresponding *N*-Boc-protected derivative is commercially available from Flamma Spa (Italy).
**(2S,4S)-Fmoc-4-Amino-1-propanoylpyrrolidine-2-carboxylic Acid (4).** The title amino acid was prepared from compound 1 according to the four-step procedure described in Scheme 1 (70% overall yield).
**(2*S*,4*R*)-Fmoc-4-Amino-1-benzoylpyrrolidine-2-carboxylic Acid (5)**. The title amino acid was prepared from compound 3 according to the four-step procedure described in Scheme 1 (65% overall yield).
**(2S,4R)-Fmoc-4-Amino-1-propanoylpyrrolidine-2-carboxylic Acid (6)**. The title amino acid was prepared from compound 3 according to the four-step procedure described in Scheme 1 (63% overall yield).
**(2*R*,4*S*)-Fmoc-4-Amino-1-Boc-pyrrolidine-2-carboxylic Acid (9)**. The title amino acid was prepared from compound 7 according to the eight-step procedure described in Scheme 1 (39% overall yield).
**(2*R*,4*R*)-Fmoc-4-Amino-1-Boc-pyrrolidine-2-carboxylic Acid (11)**. The title amino acid was prepared from compound 7 according to the eight-step procedure described in Scheme 1 (41% overall yield).
**(2S,4S)-Fmoc-4-Amino-1-(N Boc-6-aminohexanoyl)pyrrolidine-2-carboxylic** Acid (12). The title amino acid was prepared from compound 1 according to the five-step procedure described in Scheme 1 (58% overall yield).
**(2S,4S)-Fmoc-4-Amino-1-(N Boc-6-aminohexyl)pyrrolidine-2-carboxylic** Acid (13). The title amino acid was prepared from compound 1 according to the five-step procedure described in Scheme 1 (61 % overall yield).
**(2*S*,4*S*)-Fmoc-4-Amino-1-[(2-(*N*-Boc-2-aminoethoxy)ethoxy)acetyl]pyrrolidine-2-carboxylic Acid (14).** The title amino acid was prepared from compound 1 according to the five-step procedure described in Scheme 1 (64% overall yield).

### PREPARATION OF AMPRO-CONTAINING RGD CYCLOPEPTIDES (SCHEMES 2 AND 3)

### EXAMPLE 1

### Preparation of Compound (Ie) c[-Arg-Gly-Asp-AMPRO1-] (General Procedure for Cyclopeptide Synthesis).

*Resin Loading.* All number of equivalents of reagents are given relative to the resin loading (mmol/g). In a solid phase reaction vessel, to the cTrt resin (178 mg, 0.276 mmol) preswollen in CH₂Cl₂ (30 min) a solution of Fmoc-Gly-OH (206 mg, 0.69 mmol) and DIEA (110 µL, 0.635 mmol) in CH₂Cl₂ (2.5 mL) was added. The reaction mixture was stirred under a flow of nitrogen for 1 h. After adding another 110 µL of DIEA (0.635 mmol) and 500 µL of MeOH, the mixture was shaken for a further 30 min, then drained and the resin washed several times with DMF (3 × 3 mL), CH₂Cl₂ (5 × 3 mL), Pr*ⁱ*OH (2 x 3 mL), MeOH (5 x 3 mL), Et₂O (2 x 3 mL), CH₂Cl₂ (3 × 3 mL). The Fmoc-Gly resin, swollen in DMF (5 x 3 mL), was treated with 5% v/v piperidine in DMF/CH₂Cl₂ 1:1 (5 mL, 5 min). The solution was drained and the resin treated with 20% v/v piperidine in DMF (5 mL x 5 min x 6 cycles). The resin was washed with DMF (3 x 3 mL), Pr*ⁱ*OH (3 x 3 mL), Et₂O (3 x 3 mL), CH₂CI₂ (3 x 3 mL), and DMF (2 x 3 mL) and the presence of the free amino groups checked with TNBS test.

*Peptide coupling.* A preformed solution of Fmoc-Arg(Pmc)-OH (457 mg, 0.69 mmol), TBTU (176 mg, 0.55 mmol), HOBt (93 mg, 0.69 mmol) and DIEA (241 µL, 1.38 mmol) in NMP (2.5 mL) was added to the deprotected peptidyl resin. The mixture was shaken at room temperature for 1.5 h. Reaction completion was checked with TNBS test. The solution was drained and the resin washed several times with DMF (3×3 mL), Pr*ⁱ*OH (3 x 3 mL), Et₂O (2 x 3 mL), CH₂CI₂ (3 x 3 mL). The resin was washed again with DMF (5 x 3 mL) and then treated with 20% v/v piperidine in DMF (5 mL x 5 min x 6 cycles). The solution was drained and the resin washed with DMF (5 × 3 mL), Pr*ⁱ*OH (3 x 3 mL), Et₂O (2 × 3 mL), CH₂Cl₂ (2 x 3 mL), and DMF (2 × 3 mL) and the presence of the free amino groups checked with TNBS test.

The coupling of the Fmoc-**AMPRO1**-OH (250 mg, 0.55 mmol) and Fmoc-Asp(Bu*^{t}*)-OH (283 mg, 0.69 mmol) residues was carried out under the same conditions.

*Resin Cleavage.* The resin-bound peptide, H-Asp(Bu*^{t}*)-**AMPRO1**-Arg(Pmc)-Gly-O-cTrt, was treated with 5 mL of a mixture of hexafluoroisopropanol (HFIP) and CH₂Cl₂ (1:4) for 15 min at ambient temperature. The solution was recovered and the resin carefully washed with the above HFIP/CH₂Cl₂ mixture (2 x 5 mL x 10 min). The combined solution was co-evaporated under vacuum with hexane several times furnishing 92 mg of linear tetrapeptide H-Asp(Bu*^{t}*)-**AMPRO1**-Arg(Pmc)-Gly-OH as a white solid, used as such in the subsequent synthesis step.

*Cyclization. Method A (Preferred Procedure for (Ie),* (*If*), (*Ig*), (*Ik*), *and (Il)).* The linear tetrapeptide, H-Asp(Bu*^{t}*)-**AMPRO1**-Arg(Pmc)-Gly-OH (92 mg, 0.10 mmol) was dissolved in DMF (42 mL, 2.5 mM) under nitrogen and the solution was cooled at 0°C. Solid NaHC0₃ (44 mg, 0.52 mmol) and diphenylphosphoryl azide (DPPA, 67 µL, 0.31 mmol) were added, and the reaction mixture was stirred at ambient temperature for 36 h. After filtration of excess solid NaHCO₃, the mixture was diluted with water and evaporated in vacuo. The solid residue was purified by flash chromatography (EtOAc/MeOH 8:2) furnishing the protected cyclic tetrapeptide (82 mg, 95%) as a glassy white solid.

*Cyclization. Method B (Preferred Procedure for (Ih) and (Ii)).* The linear tetrapeptide, H-Asp(Bu*^{t}*)-**AMPRO1**-Arg(Pmc)-Gly-OH (92 mg, 0.10 mmol) was dissolved in DMF (25 mL) under nitrogen at room temperature. HATU (114 mg, 0.30 mmol), HOAt (42 µL, 0.30 mmol) and 2,4,6-collidine (40 mg, 0.30 mmol) were added, and the reaction mixture was stirred at ambient temperature for 12 h. The reaction mixture was evaporated under vacuum, the solid residue dissolved in EtOAc (5 mL) and the solution was washed with 5% aqueous NaHCO₃ The organic layers were collected, dried over MgSO₄, filtered, and evaporated under vacuum to afford a crude residue which was purified by flash chromatography (EtOAc/MeOH 8:2) furnishing the protected cyclic tetrapeptide (65 mg, 75%) as a glassy white solid.

*Side Chain Deprotection.* The protected cyclic tetrapeptide (82 mg, 0.095 mmol) was treated with 5 mL of a solution of TFA (85.5%), phenol (5%), water (2%), thioanisole (5%), and 1,2-ethanedithiol (2.5%) at ambient temperature. After 24 h, the solvent was evaporated under vacuum and the residue dissolved in 5 mL of 50% aq AcOH. The mixture was diluted with 5 mL of Et₂O and extracted with water (3 x 5 mL). The combined aqueous layers were washed with Et₂O (3 x 3 mL) and concentrated in vacuo. The crude residue was dissolved in 5 mL of 3N aq HCl and washed again with Et₂O (3 x 3 mL). The aqueous phase was concentrated under vacuum furnishing the deprotected cyclic tetrapeptide, c[-Arg-Gly-Asp-**AMPRO1**-] (Ie), (46 mg, quant., corresponding to an overall yield of 35%) as a hydrochloride salt.

*Peptide Purification.* The cyclic tetrapeptide was purified by semipreparative RP-HPLC (RP C18-10µm, 250 x 10 mm) using acetonitrile (0.05% TFA) in H₂O (0.05% TFA), 0-35% linear gradient over 25 min. A flow rate of 5.0 mL/min was used and detection was at 254 nm. HPLC Rₜ = 5.8 min. Purity of final cyclopeptide was checked with analytical HPLC (Discovery C18-10µm column, 250 x 4.6 mm) in two different solvent systems (methanol/water and acetonitrile/water) using a gradient program and found to be ≥98% pure. The HPLC sample was evaporated under vacuum, and finally transformed into the hydrochloride salt by exposing the solid material to anhydrous gaseous HCl until constant weight was reached, ready for biological assay. A white solid; [α]_{D}²⁵ -5.80 (c 0.62, H₂O) (HCl salt); ¹H NMR (800 MHz, H₂O/D₂O 90:10, 298K) δ 9.07 (NH Gly), 8.90 (NH Arg), 8.51 (NH Asp), 7.30 (NH AMPRO), 7.20 (NHε Arg), 4.73 (Hα Asp), 4.67 (H2 AMPRO), 4.51 (H4 AMPRO), 4.25 (Hα Arg), 4.13 and 3.56 (Hα Gly), 3.72 and 3.68 (H5 AMPRO), 3.24 (Hδ Arg), 2.94 (Hβ Asp), 2.77 and 2.48 (H3 AMPRO), 1.79 and 1.73 (Hβ Arg), 1.73 and 1.65 (Hγ Arg); ¹³C NMR (200 MHz, H₂O/D₂O 90:10, 298K) δ 59.0 (C2 AMPRO), 58.20 (Cα Arg), 55.30 (C5 AMPRO), 52.33 (C4 AMPRO), 47.40 (Cα Gly), 43.40 (Cδ Arg), 38.70 (C3 AMPRO), 37.80 (Cβ Asp), 29.30 (Cβ Arg), 27.20 (Cγ Arg). HRMS (ES+) C₁₇H₂₉N₈O₆ calcd for [MH]⁺ 441.2210, found 440.2232.

### EXAMPLE 2

*Preparation of Compound (If) c[-Arg-Gly-Asp-**AMPR02**-].* The title compound was prepared according to the procedure described for (Ie) (Example 1) and utilizing subunit Fmoc-AMPRO2-OH (250 mg, 0.548 mmol). Resin loading: 1.55 mmol/g. Overall yield: 34% (54 mg). A white solid; [α]_{D}²⁵ -8.56 (c 0.66, H₂O (HCI salt); HPLC purity: ≥98%; RP-HPLC (RP C18-10µm, 250 x 10 mm) using acetonitrile (0.05% TFA) in H₂O (0.05% TFA), 0-35% linear gradient over 25 min; flow rate 5.0 mL/min, detection at 254 nm, Rₜ = 11.8 min. ¹H NMR (800 MHz, H₂O/D₂O 90:10, 298K, 54:46 mixture of A/B atropoisomers) δ 8.94 (NH Gly_{A}), 8.86 (NH Arg_{A}), 8.76 (NH Gly_{B}), 8.46 (NH Arg_{B}), 8.31 (NH Asp_{A,B}), 7.53 (NH AMPRO_{B}), 7.50 (NH AMPRO_{A}), 7.3-7.5 (m, 4H, Ph), 7.22 (m, 1H, Ph), 7.20 (NHε Arg_{A}), 7.14 (NHε Arg_{B}), 4.82 (H2_{A,B} AMPRO), 4.68 (Hα Asp_{A,B}), 4.65 (H4 AMPRO_{B}), 4.52 (H4 AMPRO_{A}), 4.17 (Hα Arg_{A}), 4.14 and 3.56 (Hα Gly_{A}), 4.08 and 3.57 (Hα Gly_{B}), 4.06 and 3.81 (H5 AMPRO_{B}), 4.01 and 3.53 (H5 AMPRO_{A}), 3.88 (Hα Arg_{B}), 3.26 (Hδ Arg_{A}), 3.12 (Hδ Arg_{B}), 2.86 (Hβ Asp_{A,B}), 2.72 and 2.16 (H3 AMPRO_{A}), 2.70 and 2.06 (H3 AMPRO_{B}), 1.81 (Hβ Arg_{A}), 1.66 (Hγ Arg_{A}), 1.62 (Hβ Arg_{B}), 1.48 (Hγ Arg_{B}); ¹³C NMR (200 MHz, H_{z}O/D₂O 90:10, 298K) δ 129.8 (Ph), 128.6 (2C, Ph), 127.2 (2C, Ph), 54.72 (C5 AMPRO_{A}), 54.18 (Cα Arg_{A}), 53.80 (Cα Arg_{B}), 52.30 (C5 AMPRO_{B}), 48.34 (Cα Asp_{A,B}), 48.30 (C4 AMPRO_{A,B}), 43.00 (Cα Gly_{A}), 42.90 (Cα Gly_{B}), 39.22 (Cδ Arg_{A}), 39.00 (Cδ Arg_{B}), 35.30 (C3 AMPRO_{B}), 33.60 (C3 AMPRO_{A}), 33.50 (Cβ Asp_{A,B}), 25.05 (Cβ Arg_{A}), 24.70 (Cβ Arg_{B}), 23.05 (Cγ Arg_{B}), 23.00 (Cγ Arg_{A}). HRMS (ES+) C₂₄H₃₃N₈O₇ calcd for [MH]⁺ 545.2472, found 545.2488.

### EXAMPLE 3

*Preparation of Compound (Ig) c[-Arg-Gly-Asp-**AMPRO3**-].* The title compound was prepared according to the procedure described for (Ie) (Example 1) and utilizing subunit Fmoc-**AMPRO3**-OH (250 mg, 0.553 mmol). Resin loading: 1.55 mmol/g. Overall yield: 21 % (28 mg). A white solid; [α]_{D}²⁵ -20.37 (c 0.36, H₂O) (HCl salt); HPLC purity: >98%; RP-HPLC (RP C18-10µm, 250 x 10 mm) using acetonitrile (0.05% TFA) in H₂O (0.05% TFA), 0-35% linear gradient over 25 min; flow rate 5.0 mL/min, detection at 254 nm, Rₜ = 6.4 min.; ¹H NMR (800 MHz, H₂O/D₂O 90:10, 298K) δ 8.83 (NH Arg), 8.67 (NH Gly), 8.38 (NH Asp), 8.19 (NH AMPRO), 7.20 (NHε Arg), 4.70 (H2 AMPRO), 4.60 (Hα Asp), 4.46 (H4 AMPRO), 4.43 (Hα Arg), 4.09 and 3.81 (Hα Gly), 3.78 and 3.35 (H5 AMPRO), 3.22 (Hδ Arg), 2.87 (Hβ Asp), 2.50 and 2.44 (H3 AMPRO), 1.87 and 1.79 (Hβ Arg), 1.64 (Hγ Arg); ^{l3}C NMR (200 MHz, H₂O/D₂O 90:10, 298K) δ 56.37 (Cα Arg), 53.34 (Cα Asp), 52.72 (C5 AMPRO), 51.80 (C4 AMPRO), 45.57 (Cα Gly), 43.40(Cδ Arg), 38.74 (Cβ Asp), 37.26 (C3 AMPRO), 30.90 (Cβ Arg), 27.17 (Cγ Arg). HRMS (ES+) C₁₇H₂₉N₈O₆ calcd for [MH]⁺ 441.2210, found 440.2201.

### EXAMPLE 4

*Preparation of Compound (Ih) c[-Arg-Gly-Asp-**AMPRO4**-].* The title compound was prepared according to the procedure described for (Ie) (Example 1) and utilizing subunit Fmoc-**AMPRO4**-OH (204 mg, 0.50 mmol). Resin loading: 1.55 mmol/g. Overall yield: 22% (29 mg). A white solid; [α]_{D}²⁵ -14.5 (c 0.68, H₂O) (HCl salt); HPLC purity: ≥98%; RP-HPLC (RP C18-10µm, 250 x 10 mm) using acetonitrile (0.05% TFA) in H₂O (0.05% TFA), 0-25% linear gradient over 25 min; flow rate 5.0 mL/min, detection at 254 nm, Rₜ = 9.39 min.; ¹H NMR (800 MHz, H₂O/D₂O 90:10, 298K, 80:20 mixture of A/B atropoisomers) δ 8.91 (NH Gly_{B}),8.84 (NH Gly_{A}), 8.80 (NH Arg_{B}), 8.70 (NH Arg_{A}), 8.26 (NH Asp_{A,B}), 7.38 (NH AMPRO_{A}), 7.30 (NH AMPRO_{B}), 7.13 (NHε Arg_{A,B}), 4.67 (Hα Asp_{A,B}), 4.59 (H2 AMPRO_{A}), 4.50 (H4 AMPRO_{A}), 4.42 (H4 AMPRO_{B}), 4.11 (Hα Arg_{B}), 4.10 and 3.48 (Hα Gly_{B}), 4.08 and 3.49 (Hα Gly_{A}), 4.07 (Hα Arg_{A}), 3.88 and 3.63 (H5 AMPRO_{A}), 3.77 and 3.55 (H5 AMPRO_{B}), 3.18 (Hδ Arg_{A,B}), 2.84 (Hβ Asp_{A,B}), 2.68 and 2.18 (H3 AMPRO_{B}), 2.54 and 2.04 (H3 AMPRO_{A}), 2.24 (C*H*₂CH₃), 1.73 (Hβ Arg_{A,B}), 1.58 (Hγ Arg_{A}), 1.57 (Hγ Arg_{B}), 0.90 (CH₂*CH*_{3A}), 0.88 (CH₂*CH*_{3B}); ¹³C NMR (200 MHz, H₂O/D₂O 90:10, 298K) δ 56.60 (C5 AMPRO_{B}), 56.40 (C5 AMPRO_{A}), 47.22 (Cα Gly_{A,B}), 43.47 (Cδ Arg_{A,B}), 39.53 (C3 AMPRO_{B}), 37.77 (Cβ Asp_{A,B}), 37.63 (C3 AMPRO_{A}), 29.19 (Cβ Arg_{A,B}), 27.50 (CH₂CH₃), 27.23 (Cγ Arg_{A,B}), 10.30 (CH₂CH₃). HRMS (ES+) C₂₀H₃₃N₈O₇ calcd for [MH]⁺ 497.2472, found 497.2460.

### EXAMPLE 5

*Preparation of Compound (Ii) c[-Arg-Gly-Asp-**AMPRO5**-].* The title compound was prepared according to the procedure described for (Ie) (Example 1) and utilizing subunit Fmoc**-AMPRO5-**OH (250 mg, 0.548 mmol). Resin loading: 1.55 mmol/g. Overall yield: 22% (35 mg). A white solid; [α]_{D}²⁵ -22.1 (c 0.2, H₂O) (HCI salt); HPLC purity: ≥98%; RP-HPLC (RP C18-10µm, 250 x 10 mm) using acetonitrile (0.05% TFA) in H₂O (0.05% TFA), 0-25% linear gradient over 25 min; flow rate 5.0 mL/min, detection at 254 nm, Rₜ = 16.6 min. ¹H NMR (600 MHz, H₂O/D₂O 90:10, 298K mixture of atropoisomers, major isomer) δ 8.90 (NH Gly), 8.85 (NH Arg), 8.28 (NH Asp), 7.54 (NH AMPRO), 7.3-7.5 (m, 5H, Ph), 7.20 (NHε Arg), 4.73 (H2 AMPRO), 4.38 (Hα Arg), 4.34 (Hα Asp), 4.24 (H4 AMPRO), 3.85 and 3.69 (Hα Gly), 3.74 and 3.47 (H5 AMPRO), 3.04 (Hδ Arg), 2.65 (Hβ Asp), 2.35 and 2.19 (H3 AMPRO), 1.83 and 1.70 (Hβ Arg), 1.58 (Hγ Arg); ¹³C NMR (200 MHz, H₂O/D₂O 90:10, 298K, mixture of atropoisomers, major isomer) δ 129.8 (Ph), 128.6 (2C, Ph), 127.2 (2C, Ph), 55.00 (C5 AMPRO), 54.15 (Cα Arg), 48.44 (Cα Asp), 48.30 (C4 AMPRO), 43.21 (Cα Gly), 39.55 (Cδ Arg), 35.42 (C3 AMPRO), 33.50 (Cβ Asp), 25.05 (Cβ Arg), 23.00 (Cγ Arg). HRMS (ES+) C₂₀H₃₃N₈O₇ calcd for [MH]⁺ 497.2472, found 497.2484.

### EXAMPLE 6

*Preparation of Compound (Ik) c[-Arg-Gly-Asp-**AMPRO9**-].* The title compound was prepared according to the procedure described for (Ie) (Example 1) and utilizing subunit Fmoc-**AMPRO9**-OH (250 mg, 0.55 mmol). Resin loading: 1.55 mmol/g. Overall yield: 34% (45 mg). A white solid; [α]_{D}²⁵ -3.6 (c 0.3, H₂O) (HCl salt); HPLC purity: ≥98%; RP-HPLC (RP C18-10µm, 250 x 10 mm) using acetonitrile (0.05% TFA) in H₂O (0.05% TFA), 0-25% linear gradient over 25 min; flow rate 5.0 mL/min, detection at 220 nm, Rₜ = 7.5 min.; ¹H NMR (800 MHz, H₂O/D₂O 90:10, 298K) δ 8.82 (NH Arg), 8.65 (NH Gly), 8.40 (NH Asp), 8.15 (NH AMPRO), 7.18 (NHε Arg), 4.54 (H2 AMPRO), 4.51 (Hα Asp), 4.39 (H4 AMPRO), 4.26 (Hα Arg), 3.89 and 3.75 (Hα Gly), 3.62 and 3.29 (H5 AMPRO), 3.07 (Hδ Arg), 2.71 (Hβ Asp), 2.39 and 2.28 (H3 AMPRO), 1.75 and 1.64 (Hβ Arg), 1.49 (Hγ Arg); ¹³C NMR (200 MHz, H_{Z}O/D₂O 90:10, 298K) δ 56.37 (Cα Arg), 53.34 (Cα Asp), 52.72 (C5 AMPRO), 51.80 (C4 AMPRO), 45.57 (Cα Gly), 43.40(Cδ Arg), 38.74 (Cβ Asp), 37.26 (C3 AMPRO), 30.90 (Cβ Arg), 27.17 (Cγ Arg). HRMS (ES+) C₁₇H₂₉N₈O₆ calcd for [MH]⁺ 441.2210, found 440.2230.

### EXAMPLE 7

*Preparation of Compound (Il) c[-Arg-Gly-Asp-**AMPRO11**-].* The title compound was prepared according to the procedure described for (Ie) (Example 1) and utilizing subunit Fmoc-**AMPRO11**-OH (250 mg, 0.55 mmol). Resin loading: 1.55 mmol/g. Overall yield: 38% (50 mg). A white solid; [α]_{D}²⁵ +4.5 (c 0.4, H₂O) (HCI salt); HPLC purity: ≥98%; RP-HPLC (RP C18-10µm, 250 x 10 mm) using acetonitrile (0.05% TFA) in H₂O (0.05% TFA), 0-25% linear gradient over 25 min; flow rate 5.0 mL/min, detection at 220 nm, Rₜ = 6.2 min. ¹H NMR (800 MHz, H₂O/D₂O 90:10, 298K) δ 9.05 (NH Gly), 8.88 (NH Arg), 8.56 (NH Asp), 7.31 (NH AMPRO), 7.20 (NHε Arg), 4.67 (Hα Asp), 4.55 (H2 AMPRO), 4.31 (H4 AMPRO), 3.98 and 3.57 (Hα Gly), 3.74 (Hα Arg), 3.64 and 3.43 (H5 AMPRO), 3.09 (Hδ Arg), 2.88 and 2.69 (Hβ Asp), 2.58 and 2.35 (H3 AMPRO), 2.00 and 1.83 (Hβ Arg), 1.56 and 1.50 (Hγ Arg); ¹³C NMR (200 MHz, H₂O/D₂O 90:10, 298K) δ 58.8 (C2 AMPRO), 58.10 (Cα Arg), 55.25 (C5 AMPRO), 52.30 (C4 AMPRO), 47.42 (Cα Gly), 43.35 (Cδ Arg), 38.74 (C3 AMPRO), 37.81 (Cβ Asp), 29.36 (Cβ Arg), 27.00 (Cγ Arg). HRMS (ES+) C₁₇H₂₉N₈O₆ calcd for [MH]⁺ 440.2210, found 440.2218.

### SOLID-PHASE RECEPTOR-BINDING ASSAY (TABLE 1)

### EXAMPLE 8

*Solid-Phase Receptor-Binding Assay (General Procedure).* The receptor binding assays were performed as described previously (Kumar, C. C.; et al. J. Pharmacol. Exp. Ther. 1997, 283, 843). Purified αᵥβ₃ and αᵥβ₅ receptors (Chemicon International Inc., Temecula, CA) were diluted to 500 ng/mL and 1000 ng/mL, respectively, in coating buffer [20 mM Tris-HCl (pH 7.4), 150 mM NaCl, 2 mM CaCl₂, 1 mM MgCl₂ and 1 mM MnCl₂]. An aliquot of diluted receptors (100 µL/well) was added to 96-well microtiter plates and incubated overnight at 4 °C. Coating solution was removed and 200 µL of blocking solution (coating buffer plus 1% BSA) were added to the wells and incubated for additional 2 h at room temperature. After incubation, the plates were rinsed three times with 200 µL of blocking-binding solution and incubated for 3 h at room temperature with 0.05 nM and 0.1 nM [¹²⁵I]Echistatin (Amersham Pharmacia) for αᵥβ₃ and αᵥβ₅ receptor binding assays, respectively. After incubation, the plates were sealed and counted in the γ-counter (Packard).

All the art and procedures disclosed in the literature cited in this description which are essential to understand and reproduce this invention are to be considered as integral part of this same description.

## Claims

1. A compound of general formula (I): 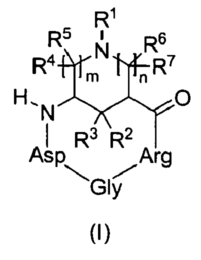 wherein
-Arg-Gly-Asp- corresponds to the sequence of naturally occurring amino acids L-Arginine, Glycine, L-Aspartic Acid;
n is 0 or 1;
m is 1 or 2;
provided that when m is 2, n is 0;
R¹ is H, alkyl, aryl, acyl, aroyl, a protective group or a divalent linking moiety;
R², R³, R⁶, R⁷ are independently H, OH, alkoxyl, aryloxyl, alkyl, aryl, acyl, aroyl;
R⁴ and R⁵ are independently H, OH, alkoxyl, aryloxyl, alkyl, aryl, acyl, aroyl, or R⁴ and R⁵ together form an oxo group;
its salts, racemic mixtures, individual enantiomers, individual diastereoisomers and mixtures thereof in any proportion.

2. The compound according to claim 1, selected among a compound of formulae (Ia), (Ib), (Ic), and (Id): 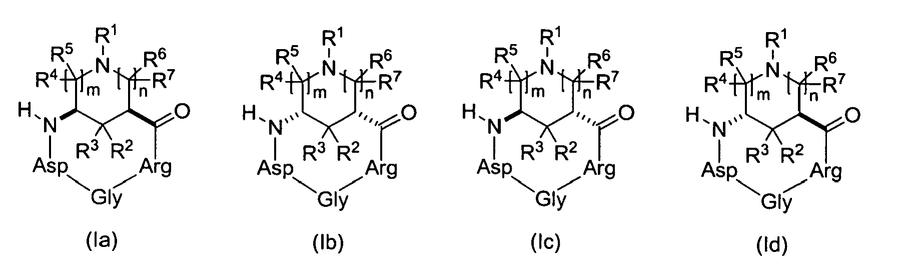 wherein Arg-Gly-Asp, n, m, R¹, R², R³, R⁴, R⁵, R⁶, and R⁷ are as defined in claim 1 and the wedge-shaped and dashed bonds indicate that the substituents are positioned above and below the plane, respectively.

3. The compound according to claims 1 or 2, **characterized in that** n is 0.

4. The compound according to claims 3, **characterized in that** m is 1 and R², R³, R⁴, and R⁵ are H.

5. The compound according to claims 1 or 2, **characterized in that** n is 0, m is 1, R¹ is H or benzoyl [(C=O)Ph] or propanoyl [(C=O)CH₂CH₃], or (CH₂)₆-NH₂, or (CH₂)₆-N₃, or (C=O)-(CH₂)₅-NH₂, or (C=O)-(CH₂)₅-N₃, or (C=O)-(CH₂OCH₂)ₚ-CH₂NH₂, or (C=O)-(CH₂OCH₂)ₚ-CH₂N₃ wherein p is preferably 3 and 4, R² is H, R³ is H, R⁴ and R⁵ are H,H or together form an oxo group.

6. The compound according to claims 1 or 2, **characterized in that** n is 0, m is 2, R¹ is H or benzoyl [(C=O)Ph] or propanoyl [(C=O)CH₂CH₃], or (CH₂)₆-NH₂, or (CH₂)₆-N₃, or (C=O)-(CH₂)₅-NH₂, or (C=O)-(CH₂)₅-N₃, or (C=O)-(CH₂OCH₂)ₚ-CH₂NH₂, or (C=O)-(CH₂OCH₂)ₚ-CH₂N₃ wherein p is preferably 3 and 4, R² is H, R³ is H, R⁴ and R⁵ are H,H or together form an oxo group.

7. The compound according to claims 1 or 2, **characterized in that** n is 1, m is 1, R¹ is H or benzoyl [(C=O)Ph] or propanoyl [(C=O)CH₂CH₃], or (CH₂)₆-NH₂, or (CH₂)₆-N₃, or (C=O)-(CH₂)₅-NH₂, or (C=O)-(CH₂)₅-N₃, or (C=O)-(CH₂OCH₂)ₚ-CH₂NH₂, or (C=O)-(CH₂OCH₂)ₚ-CH₂N₃ wherein p is preferably 3 and 4, R² is H, R³ is H, R⁴ and R⁵ are H,H or together form an oxo group, R⁶ and R⁷ are H,H or together form an oxo group.

8. The compound according to claim 1, selected among a compound of formulae (Ie), (If), (Ig), (Ih), (Ii), (Ij), (Ik), (Il), (Im), (In) and (Io)

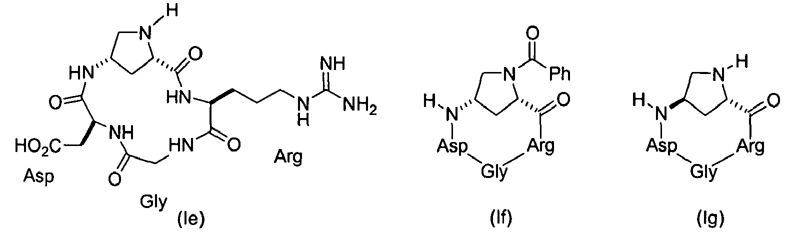

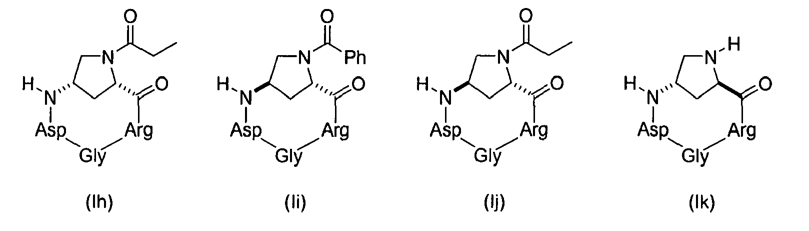

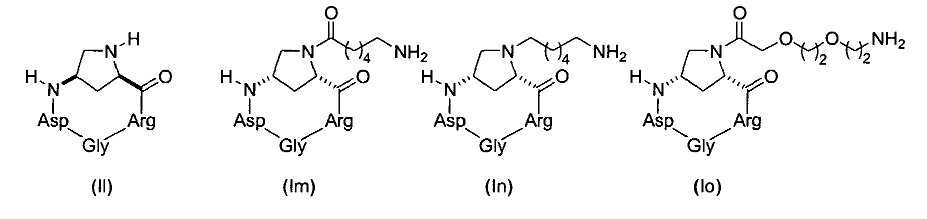

9. The compound according to claims 1 or 2, **characterized in that** R¹ is a divalent linking moiety connected to a biologically active moiety.

10. The compound according to claim 9, **characterized in that** said biologically active moiety is selected among a therapeutically or diagnostically effective molecule, a drug, an imaging detectable moiety, a chelated or polychelated complex of a paramagnetic metal ion, a chelated and polychelated complex of a radionuclide.

11. A compound according to claims 1 to 10 for its use as a medicament.

12. Use of a compound according to claims 1 to 10 for the preparation of a medicament antagonist towards αᵥβ₃ and αᵥβ₅ integrins.

13. Use of a compound according to claims 1 to 10 for the preparation of a medicament with antiangiogenic activity.

14. Use of a compound according to claims 1 to 10 for the preparation of a medicament intended for the treatment and/or the prophylaxis of altered angiogenic processes, metastasized tumour processes, retinopathies, acute renal damage and osteoporosis.

15. Use of a compound according to claims 1 to 10 as a carrier for drugs or for diagnostics.

16. A pharmaceutical composition comprising, as the active ingredient, at least one compound according to claims 1 to 10, optionally in combination with one or more pharmaceutically acceptable carriers or excipients.

17. A process for the preparation of a compound according to claims 1 to 10, **characterized by** the following schema, wherein XAA denotes the nitrogen-containing heterocyclic subunit, in particular an AMPRO subunit, or an AMPIPE subunit, or an AMNIPE subunit, 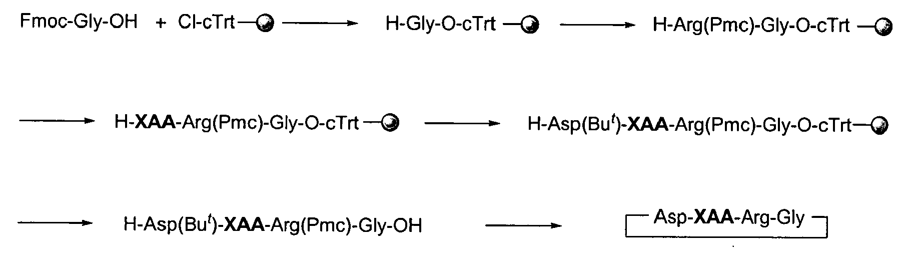 the solid phase synthesis of the resin-bound dipeptide H-Arg(Pmc)-Gly-O-c-Trt-resin according to the Fmoc strategy;
the incorporation of the pyrrolidine- or piperidine-based amino acid unit (XAA) into the above dipeptide according to conventional solid phase Fmoc strategy affording resin-bound tripeptide H-XAA-Arg(Pmc)-Gly-O-cTrt-resin;
the solid phase synthesis of the resin-bound tetrapeptide H-Asp(Bu*^{t}*)-XAA-Arg(Pmc)-Gly-O-c-Trt-resin according to the Fmoc strategy and acidic cleavage from the resin of the corresponding linear tetrapeptide H-Asp(Bu*^{t}*)-XAA-Arg(Pmc)-Gly-OH;
the solution phase macrocyclization of the above linear tetrapeptide, global deprotection, and purification to afford the target cyclopeptides of general formula
